(19) Europäisches Patentamt
European Patent Office
Office européen des brevets

(11) **EP 3 800 645 A1**

(12) **EUROPEAN PATENT APPLICATION**

(43) Date of publication:
**07.04.2021 Bulletin 2021/14**

(51) Int Cl.:
***G16H 50/20*** *(2018.01)*

(21) Application number: **19201305.0**

(22) Date of filing: **03.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
  • **PATIL, Ravindra Balasaheb**
  **5656 AE Eindhoven (NL)**

  • **RAVI, Vidya**
  **5656 AE Eindhoven (NL)**
  • **BOUMANS, Michael Leonardus Helena**
  **5656 AE Eindhoven (NL)**
  • **BUSSA, Nagaraju**
  **5656 AE Eindhoven (NL)**

(74) Representative: **Philips Intellectual Property & Standards**
**High Tech Campus 5**
**5656 AE Eindhoven (NL)**

(54) **MONITORING PERFORMANCE OF A PREDICTIVE COMPUTER-IMPLEMENTED MODEL**

(57)    According to an aspect there is provided a computer-implemented method of monitoring performance of a predictive computer-implemented model, PCIM, that is used to monitor the status of a first system. The PCIM receives as inputs observed values for a plurality of features relating to the first system, and the PCIM determines whether to issue status alerts based on the observed values. The method comprises: obtaining reference information for the PCIM, wherein the reference information for the PCIM comprises a first set of values for the plurality of features relating to the first system in a first time period; determining a set of reference probability distributions from the first set of values, the set of reference probability distributions comprising a respective reference probability distribution for each of the features that is determined from the values of the respective feature in the first set of values; obtaining operational information for the PCIM, wherein the operational information for the PCIM comprises a second set of values for the plurality of features relating to the first system in a second time period that is after the first time period; determining a set of operational probability distributions from the second set of values, the set of operational probability distributions comprising a respective operational probability distribution for each of the features that is determined from the values of the respective feature in the second set of values; determining a drift measure for the PCIM representing a measure of drift in performance of the PCIM between the first time period and the second time period, wherein the drift measure is based on a comparison of the set of reference probability distributions and the set of operational probability distributions; and output the drift measure.

Fig. 8

**Description**

FIELD OF THE INVENTION

The disclosure relates to the monitoring of the performance of a predictive computer-implemented model (PCIM) that is used to monitor the status of a system, and in particular to a computer-implemented method, apparatus and computer program product for monitoring the performance of a PCIM.

BACKGROUND OF THE INVENTION

Predictive computer-implemented models, PCIMs (also referred to as 'predictive models' and 'prediction models' herein) are becoming increasingly common across many platforms and systems where the goal is to identify possible disruptions of services or systems ahead of time and fix the issue with minimal disruption to end users of the service or system. In the context of healthcare-based imaging systems such as magnetic resonance imaging (MRI), computerised tomography (CT), Image-Guided Therapy (IGT), etc., predictive models are being built and used to alert interested parties (e.g. monitoring engineers and service engineers that are remote from the service or system) of possible system issues and failures that can be serviced ahead of failure in order to prevent and/or reduce system down time. Machine learning and statistics-based models have been developed to achieve the predictive maintenance of the system.
Typically these predictive models are built using historic data and patterns that a data scientist in conjunction with subject matter experts extract from data logs of the system. These models can determine a score for the system every day based on new incoming data, and send out alerts for systems as appropriate. However, the systems may evolve over time, e.g. there may be changes in aspects of the system hardware, and/or there can be subtle drifts or changes in usage patterns, software, firmware, etc. which can cause the accuracy of the predictive model to drift or deteriorate over time. For example, if there are changes to the structure of system data that is input into the predictive model, or if changes in software cause certain key words to change, the predictive model's performance can be affected. Of course other types of changes can occur that might affect the predictive performance of a predictive model.

SUMMARY OF THE INVENTION

While it is known that predictive models may need to be fine-tuned or corrected over a period of time so that there is no or limited deterioration of the model health (i.e. in terms of the prediction performance), there is no reliable approach or technique that can automatically monitor the predictive model and provide indications on the performance of the predictive model. Currently the monitoring of predictive models is subjective and is based on a subject matter expert (e.g. an engineer) reviewing the output of the predictive model with the past service history and system logs to assess the current health of the model.
These problems with model drift and deterioration and current approaches to evaluate the performance of predictive models have led to consideration of how to automate the monitoring of predictive models (or at least substantially reduce the need for a subject matter expert or other person to manually review the model performance). With such automated monitoring, it may be possible to identify (and also implement) appropriate corrections to the predictive model in case drift or deterioration in the performance of the predictive model is identified.
According to a first aspect, there is provided a computer-implemented method of monitoring performance of a predictive computer-implemented model, PCIM, that is used to monitor the status of a first system. The PCIM receives as inputs observed values for a plurality of features relating to the first system, and the PCIM determines whether to issue status alerts based on the observed values. The method comprises obtaining reference information for the PCIM, wherein the reference information for the PCIM comprises a first set of values for the plurality of features relating to the first system in a first time period; determining a set of reference probability distributions from the first set of values, the set of reference probability distributions comprising a respective reference probability distribution for each of the features that is determined from the values of the respective feature in the first set of values; obtaining operational information for the PCIM, wherein the operational information for the PCIM comprises a second set of values for the plurality of features relating to the first system in a second time period that is after the first time period; determining a set of operational probability distributions from the second set of values, the set of operational probability distributions comprising a respective operational probability distribution for each of the features that is determined from the values of the respective feature in the second set of values; determining a drift measure for the PCIM representing a measure of drift in performance of the PCIM between the first time period and the second time period, wherein the drift measure is based on a comparison of the set of reference probability distributions and the set of operational probability distributions; and output the drift measure. Thus the first aspect provides for the automatic monitoring of a PCIM to identify when the PCIM is no longer operating correctly based on probability distributions of values of system features.
In some embodiments the step of determining the drift measure comprises, for each feature relating to the first system, comparing one or more statistical measures for the reference probability distribution of said feature to one or more statistical measures for the operational probability distribution of said feature.

In these embodiments the step of comparing can comprise, for each feature relating to the first system and for each statistical measure, determining a distance measure for said feature and statistical measure from the value of said statistical measure for the reference probability distribution and the value of said statistical measure for the operational probability distribution.

In these embodiments the one or more statistical measures can comprise any one or more of: a mean of the probability distribution, a standard deviation of the probability distribution, a density of the probability distribution, and one or more shape parameters defining the shape of the probability distribution.

In some embodiments the first set of values for the plurality of features is a training set of values that was used to train the PCIM, and the first time period is a time period before the PCIM is monitoring the status of the first system. These embodiments have the advantage that the performance of the PCIM can be monitored in the event that values for the plurality of features during use of the PCIM are not available for analysis.

In these embodiments the reference information for the PCIM can further comprise reference performance information indicating an expected reliability of the PCIM in issuing status alerts for the first system based on the training set of values; the operational information for the PCIM can further comprise operational performance information indicating the operational reliability of the PCIM in issuing status alerts for the first system in the second time period; and the drift measure can be further based on a comparison of the reference performance information and the operational performance information.

In alternative embodiments, the first set of values for the plurality of features is a set of values obtained during use of the PCIM, and the first time period is a time period where the PCIM is monitoring the status of the first system. These embodiments have the advantage that the performance of the PCIM can be monitored based on values of the plurality of features that have occurred while the PCIM is in use, and provides a better baseline for evaluating the performance or drift of the PCIM.

In these embodiments the reference information for the PCIM can further comprise reference performance information indicating the reliability of the PCIM in issuing status alerts for the first system in the first time period; the operational information for the PCIM can further comprise operational performance information indicating the operational reliability of the PCIM in issuing status alerts for the first system in the second time period; and the drift measure can be further based on a comparison of the reference performance information and the operational performance information.

In these embodiments each of the reference performance information and the operational performance information can comprise one or more of a true positive rate, a false positive rate, a true negative rate and a false negative rate.

In some embodiments the method further comprises: obtaining values of one or more further features relating to the first system, the one or more further features comprising any of a presence of a log file for the first system, a warranty status of a component of the first system, a version of software or firmware used by the first system; and the drift measure is further based on the values of the one or more further features.

In some embodiments the method further comprises: analysing the PCIM to identify the plurality of features relating to the first system that are used by the PCIM. These embodiments have the advantage that the PCIM can be assessed to automatically identify the features that are to be used in the evaluation of the PCIM.

In some embodiments the method further comprises: evaluating the drift measure to identify one or more of the features that have contributed to the value of the drift measure; and analysing the identified one or more features that have contributed to the value of the drift measure to determine corrections to the operation of the PCIM to reduce the drift measure. These embodiments provide the advantage that the causes of the drift of the PCIM are identified and fixes to the PCIM suggested to correct the drift.

In some embodiments the method further comprises: analysing the determined drift measure to estimate a remaining life of the PCIM.

According to a second aspect, there is provided a computer program product comprising a computer readable medium having computer readable code embodied therein, the computer readable code being configured such that, on execution by a suitable computer or processor, the computer or processor is caused to perform the method according to the first aspect or any embodiment thereof.

According to a third aspect, there is provided an apparatus for monitoring performance of a PCIM that is used to monitor the status of a first system. The PCIM receives as inputs observed values for a plurality of features relating to the first system, and the PCIM determines whether to issue status alerts based on the observed values. The apparatus comprises a processing unit is configured to: obtain reference information for the PCIM, wherein the reference information for the PCIM comprises a first set of values for the plurality of features relating to the first system in a first time period; determine a set of reference probability distributions from the first set of values, the set of reference probability distributions comprising a respective reference probability distribution for each of the features that is determined from the values of the respective feature in the first set of values; obtain operational information for the PCIM, wherein the operational information for the PCIM comprises a second set of values for the plurality of features relating to the first system in a second time period that is after the first time period; determine a set of operational probability distributions from the second set of values, the set of operational probability distributions comprising a respective operational probability distribution for each of the features that is determined from the values of the respective feature in the second set of values; determine a drift measure for the PCIM representing a measure of drift in performance of the PCIM between the first time period and the second time period, wherein the drift measure is based on a comparison of the set of reference probability distributions and the set of operational probability distributions; and cause the output of the drift measure. Thus the third aspect provides for the automatic monitoring of a PCIM to identify when the PCIM is no longer operating correctly based on probability distributions of values of system features.

In some embodiments the processing unit is configured to determine the drift measure by, for each feature relating to the first system, comparing one or more statistical measures for the reference probability distribution of said feature to one or more statistical measures for the operational probability distribution of said feature.

In these embodiments the processing unit can be configured to compare, for each feature relating to the first system and for each statistical measure, by determining a distance measure for said feature and statistical measure from the value of said statistical measure for the reference probability distribution and the value of said statistical measure for the operational probability distribution.

In these embodiments the one or more statistical measures can comprise any one or more of: a mean of the probability distribution, a standard deviation of the probability distribution, a density of the probability distribution, and one or more shape parameters defining the shape of the probability distribution.

In some embodiments the first set of values for the plurality of features is a training set of values that was used to train the PCIM, and the first time period is a time period before the PCIM is monitoring the status of the first system. These embodiments have the advantage that the performance of the PCIM can be monitored in the event that values for the plurality of features during use of the PCIM are not available for analysis.

In these embodiments the reference information for the PCIM can further comprise reference performance information indicating an expected reliability of the PCIM in issuing status alerts for the first system based on the training set of values; the operational information for the PCIM can further comprise operational performance information indicating the operational reliability of the PCIM in issuing status alerts for the first system in the second time period; and the drift measure can be further based on a comparison of the reference performance information and the operational performance information.

In alternative embodiments, the first set of values for the plurality of features is a set of values obtained during use of the PCIM, and the first time period is a time period where the PCIM is monitoring the status of the first system. These embodiments have the advantage that the performance of the PCIM can be monitored based on values of the plurality of features that have occurred while the PCIM is in use, and provides a better baseline for evaluating the performance or drift of the PCIM.

In these embodiments the reference information for the PCIM can further comprise reference performance information indicating the reliability of the PCIM in issuing status alerts for the first system in the first time period; the operational information for the PCIM can further comprise operational performance information indicating the operational reliability of the PCIM in issuing status alerts for the first system in the second time period; and the drift measure can be further based on a comparison of the reference performance information and the operational performance information.

In these embodiments each of the reference performance information and the operational performance information can comprise one or more of a true positive rate, a false positive rate, a true negative rate and a false negative rate.

In some embodiments the processing unit is further configured to: obtain values of one or more further features relating to the first system, the one or more further features comprising any of a presence of a log file for the first system, a warranty status of a component of the first system, a version of software or firmware used by the first system; and the drift measure is further based on the values of the one or more further features.

In some embodiments the processing unit is further configured to: analyse the PCIM to identify the plurality of features relating to the first system that are used by the PCIM. These embodiments have the advantage that the PCIM can be assessed to automatically identify the features that are to be used in the evaluation of the PCIM.

In some embodiments the processing unit is configured to: evaluate the drift measure to identify one or more of the features that have contributed to the value of the drift measure; and analyse the identified one or more features that have contributed to the value of the drift measure to determine corrections to the operation of the PCIM to reduce the drift measure. These embodiments provide the advantage that the causes of the drift of the PCIM are identified and fixes to the PCIM suggested to correct the drift.

In some embodiments the processing unit is further configured to: analyse the determined drift measure to estimate a remaining life of the PCIM.

These and other aspects of the invention will be apparent from and elucidated with reference to the embodiment(s) described hereinafter.

BRIEF DESCRIPTION OF THE DRAWINGS

Exemplary embodiments will now be described, by way of example only, with reference to the following drawings, in which:

Fig. 1 illustrates the general principles involved in the use of predictive models in the monitoring and maintenance of a system;
Fig. 2 is a block diagram illustrating an apparatus according to an exemplary embodiment;
Fig. 3 is a block diagram showing a PCIM monitoring model and the different types of information or data that can be used by the PCIM monitoring model to monitor the performance of a PCIM according to various embodiments;
Fig. 4 is a flow diagram providing a high level view of the process of monitoring of a PCIM according to various embodiments;
Fig. 5 illustrates a method of monitoring of a PCIM according to various embodiments;
Fig. 6 illustrates a method of monitoring of a PCIM according to various embodiments;
Fig. 7 shows various inputs that can be used to determine the drift of a PCIM in block 56 of Fig. 4 and step 404 of Fig. 5; and
Fig. 8 is a flow chart illustrating a general method of monitoring the performance of a PCIM according to various embodiments.

DETAILED DESCRIPTION OF EMBODIMENTS

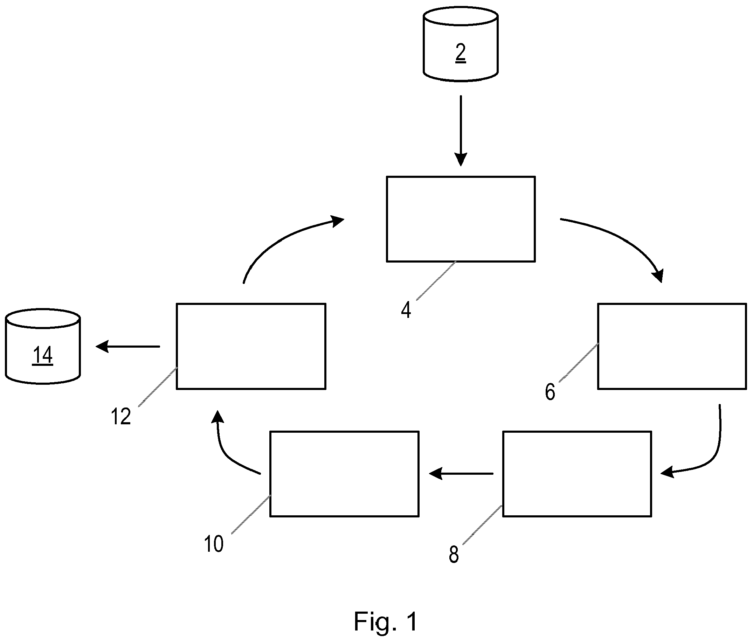

Fig. 1 illustrates the general principles involved in the creation and use of predictive models (PCIMs) in the monitoring and maintenance of a system. Any type of system can be monitored using a predictive model, for example systems such as healthcare-based imaging systems including magnetic resonance imaging (MRI), computerised tomography (CT), Image-Guided Therapy (IGT), etc. For ease of understanding, in this disclosure the terms "predictive model", "prediction model", "predictive computer-implemented model" and "PCIM" all relate to the model that is used to monitor the status of a system, such as an MRI scanner or CT scanner. The PCIM can be any type of computer-implemented machine learning model, such as a support vector machine (SVM) model, a random forest model, or a logistic regression model, etc.

Data 2 is provided from or by the system, and includes values for a number of features. The features (or 'system features') can relate to various operational or functional aspects of the system, for example, error logs, measurement logs, measurements from one or more sensors, software version, firmware version, hardware components present in the system, etc. The data 2 may also include information on errors, failures and/or other issues experienced by the system. The data 2 may be obtained from multiple sources, and relates to the system over a sufficiently long period of time to enable a predictive model to be formed. For example the data 2 can relate to the system over a period of hours, days, weeks, months or years, and as such can be considered as historical data 2.

The data 2 is collated and processed in 'data processing' block 4 so as to allow data transformations to enable the predictive model to be built or created in 'model creation' block 6. The predictive model is created so that it is able to monitor (predict) the status of the system from values of the system parameters, and to issue alerts if the predictive model predicts that an alert is required. The accuracy of the predictive model is determined in 'model evaluation' block 8 using the historic data 2, for example by the predictive model making status predictions using the historic data 2. If the accuracy of the predictive model is in an acceptable range, then the predictive model can be deployed (i.e. put into use) in 'model deployment' block 10. Thus the predictive model starts monitoring the status of the system and predicting issues with the performance of the system based on new values for the system features. The predictive model may issue an alert if an issue with the system is predicted.

After some time, the performance of the predictive model can be evaluated, for example by 'scoring' the outputs of the predictive model ('model scoring' block 12). The outputs of the predictive model can be scored based on whether alerts have been issued, and whether users of the system reported an issue with the system or the system itself reported an issue. As noted above, the performance of the predictive model might not be same as that expected following 'model evaluation' block 8 (that used the historic data 2) due to drift in the predictive model and/or various other reasons, for example due to changes in the system features (e.g. values for some system features may no longer be obtainable from the system) and/or due to the system features used by the predictive model. The 'scores' (outputs) determined for the predictive model in 'model scoring' block 12 can be stored in a database 14, along with information on whether and when issues with the system arose, including those issues reported directly by a user (operator) of the system. The values of the system features that have been evaluated by the predictive model may also be stored in database 14.

In view of the performance of the predictive model potentially drifting or deteriorating over time, it is important to be able to provide a quantitative-based assessment of the performance of the predictive model. The techniques presented herein enable the performance of a predictive model to be monitored and for alerts regarding the performance of the predictive model to be issued. It will be appreciated that the techniques presented herein effectively provide a model for monitoring the predictive model (PCIM). The model that monitors the performance of the PCIM, is referred to herein as the "PCIM monitoring model" or "PMM".

In some cases, quantitative metrics of the performance of the predictive model are available, such as True Positive (TP), False Positive (FP), Late Alert (LA) and Missed Alert (MA), and these can be used to enable objective evaluation of the performance of the predictive model since they indicate a reliability of the PCIM in issuing alerts and predicting issues with the system. The True Positive (also referred to as 'Real Positive' or 'Sensitivity') is the number of correct alerts generated by the predictive model (e.g. a user (e.g. customer) of the system called for engineer support 15 days after the predictive model issued an alert about the system). The False Positive is the number of alerts incorrectly issued by the predictive model, e.g. the predictive model issued an alert, but there was no request or complaint raised by a user of the system. The Missed Alert is the number of times that a request or complaint was raised by a user of the system but the predictive model did not (and did not subsequently) generate an alert. Finally, the Late Alert is the number of times that a request or complaint was raised by a user of the system before an alert was issued, but the predictive model subsequently generated an alert. These quantitative metrics (which are referred to herein as "performance information") may be determined and stored to enable assessment of the performance of the PCIM. In particular these quantitative metrics may be determined from information on actual alerts issued by the PCIM, and actual alerts issued by a user of the system or PCIM (e.g. a service request by an operator of the system, the logging of a fault with the system by an operator, etc.). If over time the rate of TPs by the PCIM decreases and/or the rates of FPs, LAs and/or MAs increase, then this can indicate that the performance of the PCIM has drifted and some action may need to be taken in respect of the PCIM.

However, direct information on the performance of the predictive model (i.e. the rates of TPs, FPs, LAs, MAs) may not be readily available, and so the techniques provided herein enable suitable information about the PCIM to be obtained and the PCIM to be assessed to determine if the performance of the PCIM has drifted. Optionally this assessment can be done in conjunction with performance information such as TPs, FPs, LAs and MAs.

Thus the PMM monitors the performance of the PCIM using a quantitative approach, which means that subjective bias can be removed from the decision making process about the performance of the PCIM, aiding quicker deployment of production-ready stable PCIMs. In various embodiments, the PMM can provide information about degradation of the PCIM, including an estimate of when the PCIM may need to be decommissioned and replaced, and indicate suitable corrections or adjustments that need to be performed to the PCIM to improve performance.

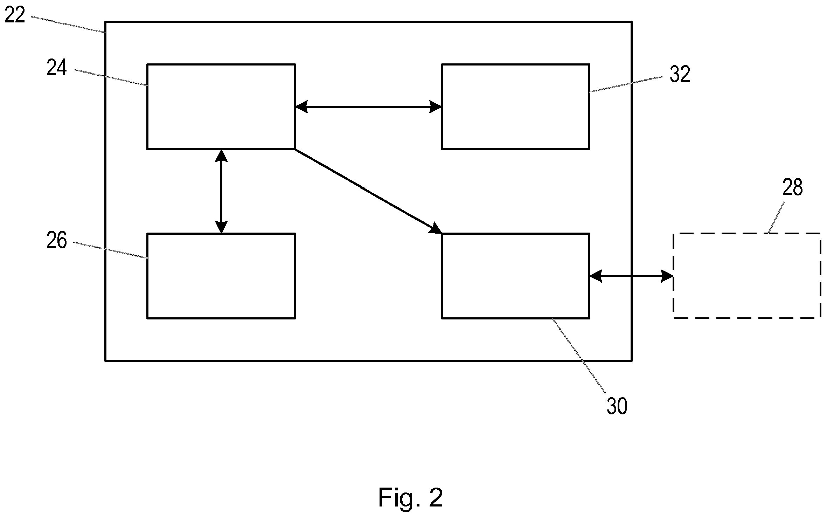

Before describing the techniques in more detail, an apparatus 22 is presented in Fig. 2 that can be used to implement various embodiments of the techniques described herein, and that in particular can be used to monitor the performance of a predictive computer-implemented model (PCIM). In some embodiments the apparatus 22 can also implement the PCIM, i.e. the apparatus 22 can receive observed values for a plurality of system parameters relating to the system, evaluate the values using the PCIM and output a status alert for the system as required.

The apparatus 22 is an electronic (e.g. computing) device that comprises a processing unit 24 and a memory unit 26. The processing unit 24 is configured or adapted to control the operation of the apparatus 22 and to implement the techniques described herein for monitoring the performance of a PCIM.

The processing unit 24 can be configured to execute or perform the methods described herein. The processing unit 24 can be implemented in numerous ways, with software and/or hardware, to perform the various functions described herein. The processing unit 24 may comprise one or more microprocessors or digital signal processor (DSPs) that may be programmed using software or computer program code to perform the required functions and/or to control components of the processing unit 24 to effect the required functions. The processing unit 24 may be implemented as a combination of dedicated hardware to perform some functions (e.g. amplifiers, pre-amplifiers, analog-to-digital convertors (ADCs) and/or digital-to-analog convertors (DACs)) and a processor (e.g., one or more programmed microprocessors, controllers, DSPs and associated circuitry) to perform other functions. Examples of components that may be employed in various embodiments of the present disclosure include, but are not limited to, conventional microprocessors, DSPs, application specific integrated circuits (ASICs), and field-programmable gate arrays (FPGAs).

The processing unit 24 is connected to a memory unit 26 that can store data, information and/or signals for use by the processing unit 24 in controlling the operation of the apparatus 22 and/or in executing or performing the methods described herein. In some implementations the memory unit 26 stores computer-readable code that can be executed by the processing unit 24 so that the processing unit 24, in conjunction with the memory unit 26, performs one or more functions, including the methods described herein. The memory unit 26 can comprise any type of non-transitory machine-readable medium, such as cache or system memory including volatile and non-volatile computer memory such as random access memory (RAM), static RAM (SRAM), dynamic RAM (DRAM), read-only memory (ROM), programmable ROM (PROM), erasable PROM (EPROM), and electrically erasable PROM (EEPROM), and the memory unit 26 can be implemented in the form of a memory chip, an optical disk (such as a compact disc (CD), a digital versatile disc (DVD) or a Blu-Ray disc), a hard disk, a tape storage solution, or a solid state device, including a memory stick, a solid state drive (SSD), a memory card, etc.

In some embodiments or implementations, the memory unit 26 stores all data required for the techniques described herein to be performed. In alternative embodiments, some or all of the data required for the techniques described herein is stored in a database or data storage unit 28 that is separate from the apparatus 22. In this case, the apparatus 22, and specifically the processing unit 24 can access the data stored in the data storage unit 28 using interface circuitry 30.

In some embodiments or implementations, the memory unit 26 and/or data storage unit 28 can store the historical data 2 that was used to generate or train the PCIM. In some embodiments and implementations the memory unit 26 and/or data storage unit 28 can store the database 14 that includes the 'scores' determined for the predictive model in 'model scoring' block 12, along with information on whether and when issues with the system arose, including those issues reported directly by a user of the system and optionally the values of the system features that have been evaluated by the predictive model.

The interface circuitry 30 is for enabling a data connection to and/or data exchange with other devices, including any one or more of servers, databases (e.g. data storage unit 28), user devices, the system that the predictive model monitors, one or more sensors that obtain values over time for the plurality of system parameters relating to the system. The connection may be direct or indirect (e.g. via the Internet), and thus the interface circuitry 30 can enable a connection between the apparatus 22 and a network, such as the Internet, via any desirable wired or wireless communication protocol. For example, the interface circuitry 30 can operate using WiFi, Bluetooth, Zigbee, or any cellular communication protocol (including but not limited to Global System for Mobile Communications (GSM), Universal Mobile Telecommunications System (UMTS), Long Term Evolution (LTE), LTE-Advanced, etc.). In the case of a wireless connection, the interface circuitry 30 (and thus apparatus 22) may include one or more suitable antennas for transmitting/receiving over a transmission medium (e.g. the air). Alternatively, in the case of a wireless connection, the interface circuitry 30 may include means (e.g. a connector or plug) to enable the interface circuitry 30 to be connected to one or more suitable antennas external to the apparatus 22 for transmitting/receiving over a transmission medium (e.g. the air). The interface circuitry 30 is connected to the processing unit 24 to enable information or data received by the interface circuitry 30 to be provided to the processing unit 24, and/or information or data from the processing unit 24 to be transmitted by the interface circuitry 30 (for example an indication of the performance of the predictive model).

In some embodiments, the apparatus 22 comprises a user interface 32 that includes one or more components that enables a user of apparatus 22 (e.g. an engineer for the system being monitored by the predictive model) to input information, data and/or commands into the apparatus 22, and/or enables the apparatus 22 to output information or data to the user of the apparatus 22. The user interface 32 can comprise any suitable input component(s), including but not limited to a keyboard, keypad, one or more buttons, switches or dials, a mouse, a track pad, a touchscreen, a stylus, a camera, a microphone, etc., and/or the user interface 32 can comprise any suitable output component(s), including but not limited to a display screen, one or more lights or light elements, one or more loudspeakers, a vibrating element, etc.

The apparatus 22 can be any type of electronic device or computing device. For example the apparatus 22 can be, or be part of, a server, a computer, a laptop, a tablet, a smartphone, a smartwatch, etc. In some implementations, the apparatus 22 is remote from the system being monitored by the predictive model. In some implementations, the apparatus 22 is remote from the apparatus or device that implements the predictive model. Alternatively, the apparatus 22 may be local to or part of the system being monitored and/or the apparatus 22 can be local to, or be, the apparatus or device that implements the predictive model.

It will be appreciated that a practical implementation of an apparatus 22 may include additional components to those shown in Fig. 2. For example the apparatus 22 may also include a power supply, such as a battery, or components for enabling the apparatus 22 to be connected to a mains power supply.

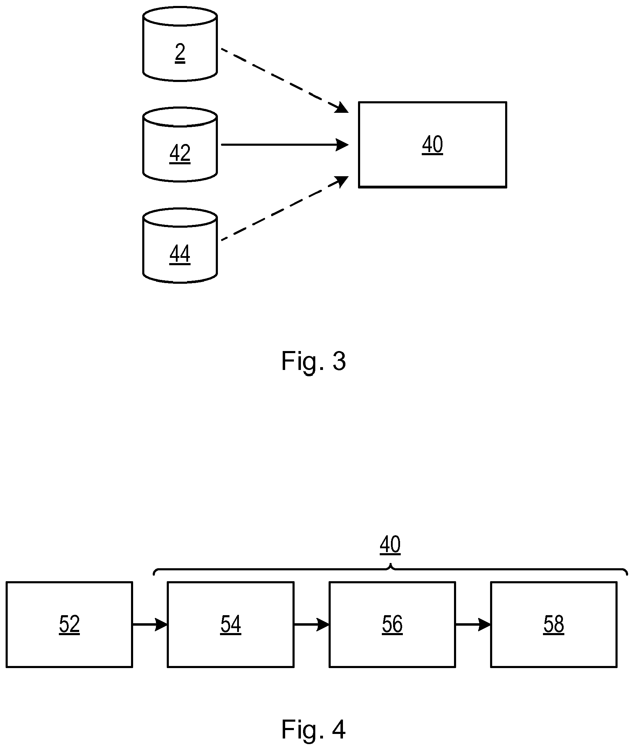

Fig. 3 is a block diagram showing a PMM and the different types of information or data that can be used by the PMM to monitor the performance of a PCIM according to various embodiments. The PMM 40 is shown as a single block, and further details of the operation of the PMM 40 will be provided below with reference to subsequent figures. In all embodiments of the techniques described herein, the PMM 40 uses a time series of values for the system features that are used by the PCIM to determine the status of the system. This time series of values is stored in operational information database 42. The time series of values for the system features can be received by the PMM 40 as they are obtained (e.g. as they are measured, and/or as they are input into the PCIM), or they can be retrieved by the PMM 40 at a later stage. In either case the time series of values for the system features in operational information database 42 relates to a time period in which the PCIM is in use and monitoring the system. The system features can relate to various operational or functional aspects of the system, for example, error logs, measurement logs, measurements from one or more sensors, software version, firmware version, hardware components present in the system, etc.

In some embodiments, the PMM 40 receives information on alerts or issues predicted by the PCIM during the period to which the time series of values stored in operational information database 42 relate. This information is stored in alert information database 44 and is referred to as "predicted alert information" or "information on predicted alerts". The information in database 44 can also or alternatively include information on alerts or issues that were raised by an operator or user of the system during the period to which the time series of values stored in database 42 relate. The information on operator or user-raised alerts or issues are referred to as "actual alert information" or "information on actual alerts" herein. The information in alert information database 44 can also or alternatively include information on the reliability of the alerts issued by the PCIM 40, e.g. in terms of a rate of TPs, FPs, LAs and/or MAs during the period to which the time series of values stored in operational data database 42 relate.

In some embodiments, the PMM 40 also makes use of the training data 2 that was used to train or create the PCIM. The training data 2 relates to values for the system features for a particular time period, and can also include information on issues with the system, such as alerts, faults, etc. and information on issues raised or flagged by an operator or user of the system, e.g. a service call, replacement of a hardware component, etc. In some embodiments, the training data 2 may also include information on the reliability that is expected from the trained PCIM in predicting issues with the system and generating alerts. This information can be any of an expected rate of TPs, FPs, LAs and/or MAs.

It will be appreciated that although the training data 2, operational information database 42 and alert information database 44 are shown as separate respective databases, any two or all of the training data 2, operational information database 42 and alert information database 44 can be stored in or on the same physical memory unit (e.g. the memory unit 26 or data storage unit 28).

Fig. 4 is a flow diagram providing a high level view of the process of monitoring of a PCIM according to embodiments of the techniques described herein. At least a part of this process can be implemented by apparatus 22. Block 52 represents the predictive model (PCIM). Information about the PCIM 52 is provided to the PMM 40, a first block of which is shown as block 54. The information about the PCIM 52 can relate to the PCIM 52 in a first time period and a second (subsequent) time period. The first time period may have any suitable length, for example, a day, several days, a week, several weeks, a month or several months. The second time period may also have any suitable length, for example, a day, several days, a week, several weeks, a month or several months.

The information about the PCIM 52 provided to block 54 relating to the second time period includes at least some of the time series of values of system features stored in operational information database 42. The time series of values for the system features in the second time period are referred to herein as "operational information", and are values of the system features relating to a more recent or most recent time period of operation of the PCIM 52. The operational information may be received by block 54 as the operational information is generated and input to the PCIM 52, or it may be retrieved from the operational information database 42 at a later time.

The information about the PCIM 52 relating to the first time period is referred to herein as "reference information". In preferred embodiments the reference information is a part of the information stored in operational information database 42 that relates to a time period earlier than the second time period, in which case the first time period is a time period in which the PCIM 52 is operational and monitoring the system. In alternative embodiments, the reference information is the training data 2, in which case the first time period is a time period before the PCIM 52 was trained and operational.

The information about the PCIM 52 provided to block 54 can also include information on errors, failures and/or other issues experienced by the system over the course of the first time period and second time period, including information on alerts issued by the PCIM 52 (i.e. the predicted alert information), information on queries or issues raised by a user of the system, e.g. the reporting of a fault by an operator of the system, the ordering of a new hardware component by an operator of the system, etc. (i.e. the actual alert information).

In block 54 the PMM 40 determines a probability distribution for each of the system features from the values of the system features in the reference information, and a probability distribution for each of the system features from the values of the system features in the operational information. The probability distributions represent the probability of observing a particular value of the respective system feature in the first/second time period respectively, and can indicate trends in the values of the system features.

The probability distributions are used by second PMM block 56 to determine if the performance of the PCIM 52 has drifted from a desired performance, in particular by comparing the probability distributions for the first time period and the second time period. This comparison provides a drift measure that indicates the amount of drift of the performance of the PCIM 52. This block 56 enables changes in the statistical properties of the values of the system features input to the PCIM 52 to be identified.

In block 58, which is optional, if a drift or sufficient drift in the PCIM 52 has been detected, the PMM 40 can determine a cause or causes of the drift (e.g. a hardware component of the system is failing, a sensor is providing inaccurate measurements, the structure or configuration of the PCIM is no longer appropriate for the current version of the system etc.), and output an indication of the cause or causes, and a recommended action for correcting the drift (e.g. replacing the hardware component, calibrating the sensor, or replacing the PCIM, etc.).

In some embodiments the predicted alert information and actual alert information can be used to assess the performance of the PCIM 52. This may be particularly useful where the statistical properties of the values of the system features input to the PCIM 52 have not substantially changed since the PCIM 52 was first created and/or deployed (e.g. if the values input to the PCIM 52 are within the same limits). From the predicted alert information and actual alert information the following metrics can be computed that are indicative of the PCIM's performance.

True positive (TP): The true positive is the number of correct alerts generated by the PCIM 52. The predicted alert is considered true if the customer or other person associated with the system (e.g. an operator or engineer) raises an alert within a 'prediction window' associated with the alert (i.e. a period of time in which the PCIM 52 predicts that the issue with the system will occur), and the raised actual alert is indicative of the same issue predicted by the PCIM 52. Also, after resolving the alert raised by the customer or other person (e.g. which can be indicated by closing a service case record in a computer system), the problem that led to the alert must have been resolved. This can be verified by checking the log files for the system and a customer alert database for the related customer alert. In other words, the TP relates to the number of predicted alerts generated by the model that map to customer calls (actual alerts) within the particular (prediction) window.

False Positive (FP): An alert predicted by the PCIM 52 is considered false if the alert raised by the PCIM 52 about the system is not followed within the prediction window by an actual alert from the customer or other user of the system.

Missed Alert (MA): Here an actual alert was raised by a customer or other user of the system but the PCIM 52 did not generate or predict a corresponding alert in the prediction window.

Late Alert (LA): Here an actual alert was raised by a customer or other user of the system but the PCIM 52 did not predict an alert until after the actual alert was raised, which prevented pre-emptive action being taken to prevent the issue from occurring or reduce the time taken to resolve the issue.

Ideally, the PCIM 52 should produce a high proportion of true positives, and a much lower proportion of missed alerts and false positives.

Thus, in certain embodiments, in order to assess the health of a predictive model 52, these metrics, along with the number of predicted alerts produced by the PCIM 52 and the consistency in providing a high volume of TPs is evaluated by the PMM 40. If there is a change in the trend of TPs, FPs, MAs and/or LAs that is out of acceptable limits, then this can be indicative of the performance of the PCIM 52 being unsatisfactory, and the PCIM 52 may need adjusting or replacing.

The trend for each PCIM 52 is unique and can be either derived by observing the PCIM 52 during its operation in monitoring the system and setting this trend as a base line for the PCIM 52, or the base line can be fixed or predetermined when the PCIM 52 is created.

When there is a decrease or continuous decrease in the number of alerts being predicted by the PCIM 52 when no decrease is expected (as in some predictive models the number of alerts decreases over time by design), there are several cases that are to be considered. Firstly, there might be issues with connectivity of the PCIM 52 to the system being monitored which can cause values of system features, such as the correct logs, to be missing from the point of view of the PCIM 52. Secondly, there may have been changes made to the software and/or firmware of the system that might have changed the wording and/or content of the logs which might cause the PCIM 52 to miss such logs. Thirdly, the system or part of the system (e.g. a hardware component) may no longer be in a warranty period and alerts in respect of that system or part are no longer relevant. Fourthly, a system that was present during the development of the PCIM 52 may be end-of-life. These factors are important and need to be assessed from time to time to ensure that the PCIM 52 is still are performing the task that it was designed to do.

Thus, the metrics mentioned above and also the probability distributions relating to the system features for the first time period need to be quantitatively measured or determined to enable the PMM 40 to take a decision on whether the performance of a PCIM 52 in monitoring the system is declining. As noted above, in some embodiments the reference information relating to the PCIM 52 can include values of system features input to the PCIM 52 during operation of the PCIM 52 in monitoring the system, and optionally also information on actual alerts and predicted alerts. In these embodiments this reference information referred to as 'ground truth' (GT) information.

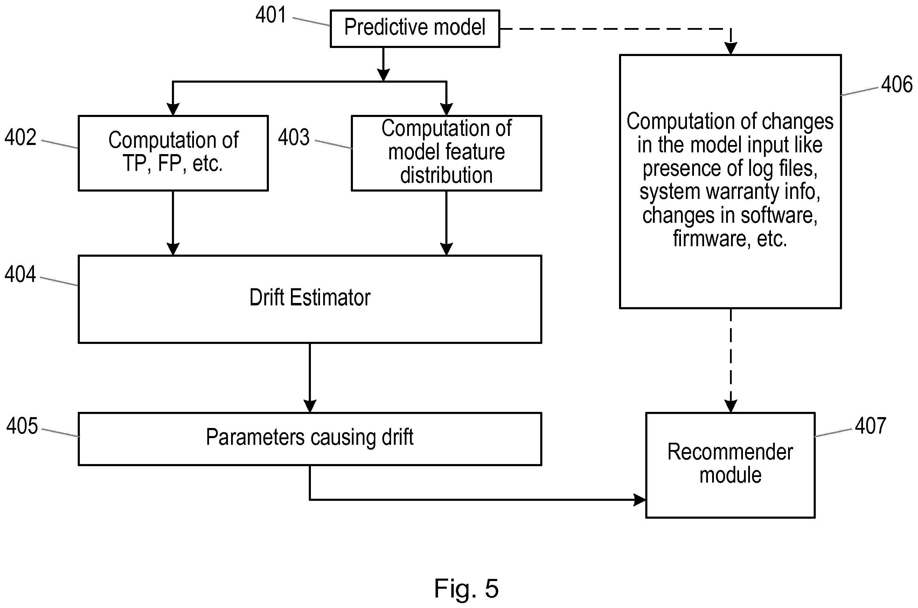

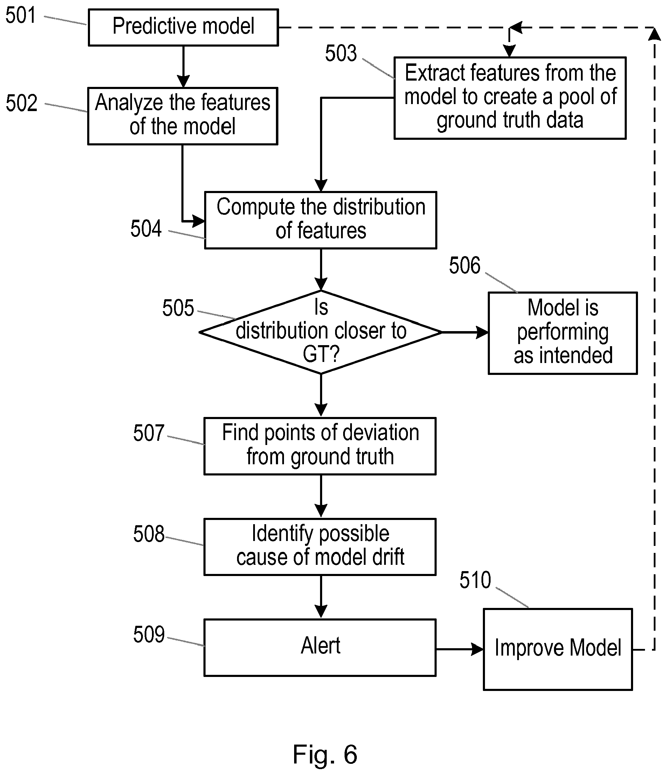

The flow chart in Fig. 5 illustrates the monitoring of a PCIM 52 according to various embodiments of the techniques described herein. The flow chart in Fig. 6 also illustrates the monitoring of a PCIM 52 according to various embodiments of the techniques described herein. The methods in Fig. 5 and Fig. 6 generally correspond to each other (although in Fig. 6 the steps are shown at a more detailed level), and generally correspond to the flow diagram in Fig. 4. One or more of the steps of the method in either of Fig. 5 or Fig. 6 can be performed by the processing unit 24 in the apparatus 22, in conjunction with any of the memory unit 26, interface circuitry 30 and user interface 32 as appropriate. The processing unit 24 may perform the one or more steps in response to executing computer program code, that can be stored on a computer readable medium, such as, for example, the memory unit 26.

Block 401 of Fig. 5 and block 501 of Fig. 6 correspond to the PCIM 52 and as such do not form a part of the illustrated method. Instead these blocks are used to indicate the steps to which the outputs of the PCIM 52 and the values of the system features are provided.

Once the PCIM 52 has been verified and deployed (block 10 of Fig. 1), the PCIM 52 starts generating alerts. These alerts contain information regarding the PCIM 52 and values of some system features for the system that support or provide the reason for this alert. It is important for the establishment of useful ground truth information to obtain values for the system features that can lead to the PCIM 52 issuing an alert about the system being monitored. Thus the PMM 40 can identify these system features or extract the names of the system features from the PCIM 52 (step 502 in Fig.

6), and the values of (at least) these system features can be obtained over a period of time (the first time period) while the PCIM 52 is deployed. The time series of values can be obtained for a sufficiently long period, such as one or more months, e.g. about 6 months, and it is assumed that the performance of the PCIM 52 is good (or at least sufficient) in the first time period. This also corresponds to step 403 in Fig. 5.

Once the ground truth information is collected, statistical measures can be determined (also in step 403 in Fig. 5 and in step 504 in Fig. 6). Examples of some statistical measures that can be determined include the mean, standard deviation and frequency (density).

Values for the metrics TP, FP, MA and LA can also be determined for the first time period. This corresponds to step 402 in Fig. 5 and step 503 in Fig. 6. These metrics represent the performance trend for the PCIM 52. In some embodiments the number of new alerts being raised each day by the PCIM 52 can be observed and an expectation of the number of new alerts can be set. For instance, there are PCIMs 52 where by design the number of alerts/day decreases over time, but for some other PCIMs 52, this might be indicative of a drift in performance of the PCIM 52.

Part of the ground truth information collection can be the computation of probability distributions for the system features (i.e. a probability distribution of the values of a system features over the ground truth data collection period) - step 403 of Fig. 5 and step 504 in Fig. 6. Statistical measures can be determined from these probability distributions.

In step 502 ground truth information is not available, and so the PCIM 52 is analysed to determine the system features used by the PCIM 52. These system features can be identified by analysing the computer file(s) that implement the PCIM 52. Values for these system features can then be obtained over the first time period.

In step 503, ground truth information is available or system features can be identified for the PCIM 52 and the identified system features used to create the GT information. In this step the information about the performance of the PCIM 52, e.g. in terms of the actual alerts vs predicted alerts, or rates of TPs, FPs, MAs and/or LAs, is available or can be computed and so the performance of the PCIM 52 can be extracted.

After the ground truth collection, the process of monitoring the PCIM 52 can start by tracking the performance of the PCIM 52 with the established ground truth information (and the ground truth pattern represented in the GT information). Significant deviation from the pattern can be understood as model drift and the next step can be to identify the source of the drift. The performance of the PCIM 52 is tracked by obtaining values of the system features when the PCIM 52 is in operation. As noted above, these values are obtained for the second time period. Probability distributions and relevant statistical measures are determined from the values obtained for the second time period.

As noted, in step 403 of Fig. 5 and step 504 of Fig. 6 probability distributions are determined for values of one or more system features, and statistical measures can be determined from these probability distributions. The probability distributions and statistical measures are determined for the values obtained for the first time period and separately for the values obtained for the second time period.

In order to find the statistical measures for a particular system feature, the time series of values the specific system feature for the relevant time period and subjected to tests that provide the best fit for the values of the system feature in terms of a probabilistic distribution. For each system feature, the distribution of values of the system feature are subjected to tests against many known types of distribution (such as beta, lognormal, normal, gamma, Weibull, etc.) and the known type of distribution with the best fit is chosen based on the QQplots. The best fit of the QQ plot is considered to be the one with the lowest least squares values between the actual data points in the distribution of the values and the fitted data points in the distribution of the values. Once the distribution has been fixed, statistical measures defining the distribution are extracted. Consider, for example, that it has been determined that values of a particular system feature follow a Beta distribution. The following computations are then performed to determine statistical measures for this system feature. The statistical measures determined from the system feature values for the first time period form a base line for the system feature.

The probability density function (PDF) for a system feature x is:

$$f(x) = \frac{\Gamma(\alpha + \beta)}{\Gamma(\alpha)\Gamma(\beta)} \frac{(x-a)^{\alpha-1}(b-x)^{\beta-1}}{(b-a)^{\alpha+\beta-1}}, \quad a \leq x \leq b, \quad a < b, \quad \alpha > 0, \quad \beta > 0 \tag{1}$$

where $\alpha$ and $\beta$ are shape parameters that are adjusted to obtain the best fit for the PDF with the distribution of values of the system feature, $\Gamma$ is the gamma function, $a$ is a lower limit of variable $x$ and $b$ is an upper limit of variable $x$.

When $a = 0$ and $b = 1$, the PDF is:

$$f(x) = \frac{\Gamma(\alpha + \beta)x^{\alpha-1}(1-x)^{\beta-1}}{\Gamma(\alpha)\Gamma(\beta)}, \quad 0 \leq x \leq 1, \quad \alpha > 0, \quad \beta > 0 \tag{2}$$

The mean is given by:

$$\text{mean} = \frac{\alpha}{\alpha + \beta} \tag{3}$$

The variance is given by:

$$\text{variance} = \frac{\alpha\beta}{(\alpha + \beta + 1)(\alpha + \beta)^2} \tag{4}$$

Similarly, for all the features of the PCIM 52, the distributions and corresponding statistical measures (such as the mean, gamma function, the values of the shape parameters, etc.) are computed and stored. When a new set of values of a system feature is received by the PMM 40 (e.g. the values of the system feature that has occurred since the PCIM 52 was put into operation), a distance metric is computed to find the deviation between the probability distributions/statistical measures of the values of the system feature for the first time period (as determined in step 403 of Fig. 5 and step 504 of Fig. 6) and the probability distributions/statistical measures for the new set of received values of the system feature. In other words, it is determined whether the probability distributions/statistical measures are similar to each other. This is performed in step 404 of Fig. 5 and corresponds to step 505 in Fig. 6.

A general distance measure can be defined as follows:

$$d_p : (y,z) \mapsto \|y-z\|_p = \left(\sum i=1n|y_i - z_i|_p\right)_{1p} \tag{5}$$

where 'y' is the PDF of the values obtained for the first time period (i.e. the ground truth information) and 'z' is the PDF of the values obtained for the second time period (i.e. when the PCIM 52 is operational). With different values for 'p', different distance metrics are computed, for example, p=1 is a Manhattan distance, p=2 is a Euclidian distance, etc.

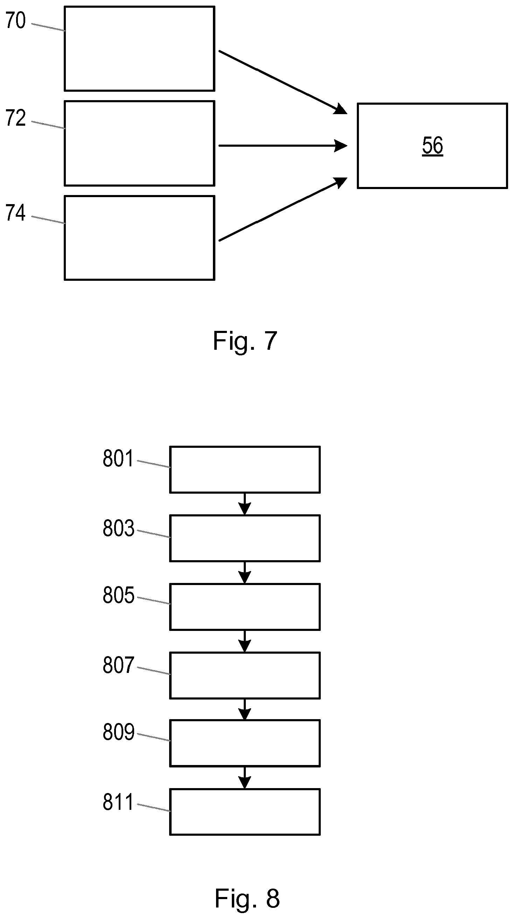

Fig. 7 shows the various inputs that can be used to determine the drift of the PCIM 52 in block 56 of Fig. 4 and step 404 of Fig. 5. The distance values $d_p$ for one or more system features (as indicated by block 70) are used to determine the drift. Optionally quantitative measures of the performance of the PCIM 52 (e.g. the TPs, FPs, LAs, and MAs) are also used to determine the drift in the performance of the PCIM 52. The quantitative measures are represented by block 72. Optionally information or values for one or more further system features can be used to determine the drift, as shown by block 74. These further system features can be a presence (or absence) of a log file for the system, a warranty status of a component of the system, a version of software or firmware used by the system, etc. These further system features can be considered as 'meta features' as they relate to slowly varying features of the system, rather than the more dynamic system features mentioned above, such as those that are obtained by one or more sensors that monitor the system. These further system features are obtained in step 406 of Fig. 5.

In block 56/step 404 the drift can be estimated or determined using a regression model which considers the above mentioned inputs as the feature values for the regression model and provides an output in the form of a drift value representing an amount of drift of the PCIM 52 from the ground truth performance. Block 56/step 404 may provide a continuous output for the drift value enabling the performance of the PCIM 52 to be continually assessed.

If in step 404 of Fig. 5 or step 505 of Fig. 6 the drift of the PCIM 52 is low (e.g. below a threshold), it can be determined that the PCIM 52 is operating or performing as intended (step 506 of Fig. 6). However, if the drift of the PCIM 52 is too high (e.g. above the threshold), then optionally a cause of the drift can be determined in step 405 of Fig. 5 and steps 507 and 508 of Fig. 6, and an action for remedying the problem with the PCIM 52 can be provided (block 58, steps 406 and 407 of Fig. 5 and steps 509 and 510 of Fig. 6). An alert can also or alternatively be issued or sent to the developer of the PCIM 52 or other relevant party indicating that the performance of the PCIM 52 is drifting. The alert may also indicate an estimate of the remaining time that the PCIM 52 can be used before it requires repair, adjustment or replacing.

Block 58/step 405/step 508 considers the drift value provided by block 56 and the values of the system feature(s) to assess the reason for the drift. In particular the system feature(s) that have drifted can be identified, and these system feature(s), or the aspect of the system that the system feature(s) relate to, can be provided as the reason(s) for the drift can be indicated. Optionally the reason for the drift can be output to the developer of the PCIM 52 or other relevant party.

In block 58/step 407/step 510 suggestions or recommendations for adjusting (e.g. fine-tuning) the one or more system features identified as the cause of the drift can be provided. These adjustments can relate to how the system feature(s) are handled by the PCIM 52. For example an adjustment can relate to how the system feature is parsed by the PCIM 52 if the issue relates to a change in a log file. In some embodiments, these adjustments can be automatically implemented by the PMM 40. For example the PMM 40 can adjust or cause the adjustment of the log file parsing structure if the log file is found to have changed. As another example, if the values of the system feature(s) are logged in a different form, the PMM 40 can rescale the values back to the original scale.

The flow chart in Fig. 8 shows a general method of monitoring the performance of a PCIM 52 according to the techniques described herein. One or more of the steps of the method in Fig. 8 can be performed by the processing unit 24 in the apparatus 22, in conjunction with any of the memory unit 26, interface circuitry 30 and user interface 32 as appropriate. The processing unit 24 may perform the one or more steps in response to executing computer program code, that can be stored on a computer readable medium, such as, for example, the memory unit 26.

In a first step, step 801, reference information is obtained for the PCIM 52. As noted above, the reference information comprises a set of values for the plurality of system features relating to the system in a first time period.

Preferably, the first time period is a time period where the PCIM 52 is deployed and monitoring the system. In that case, the set of values of the system features (the reference information) are values input into the PCIM 52 and used by the PCIM 52 to determine a status of the system. In some examples the reference information can be the values of the system features obtained during the first 6 months in which the PCIM 52 is in use, as it can be assumed that the PCIM 52 is operating correctly in this time.

In some embodiments the reference information for the PCIM further comprises reference performance information indicating the reliability of the PCIM in issuing status alerts for the first system in the first time period. The reference performance information can comprise one or more of a true positive rate, a false positive rate, a true negative rate and a false negative rate. Alternatively the reference performance information can comprise predicted alert information which is information on alerts or issues predicted by the PCIM 52 in the first time period, and actual alert information which is information on actual alerts or issues raised by a user of the system in the first time period.

In alternative embodiments, the reference information is a training set of values of system features that was used to train the PCIM 52. In this case the first time period is a time period prior to the deployment of the PCIM 52, and is a time period that spans the values of the system features in the training set. In these embodiments the reference information for the PCIM 52 can further comprise reference performance information indicating an expected reliability of the PCIM 52 in issuing status alerts for the system based on the training set of values. In other words, the reference performance information can indicate the reliability that the PCIM 52 was trained to achieve. The reference performance information can comprise one or more of a true positive rate, a false positive rate, a true negative rate and a false negative rate.

In step 803, a set of reference probability distributions are determined from the first set of values. The set of reference probability distributions comprises a respective reference probability distribution for each of the system features, and each probability distribution is determined from the values of the respective system feature in the first set of values. The probability distributions can be determined as described above with reference to step 403 of Fig. 5 and step 504 of Fig. 6.

Next, in step 805, operational information for the PCIM 52 is obtained. The operational information for the PCIM 52 comprises a set of values for the plurality of system features relating to the system in a second time period that is after the first time period. The second time period is a time period in which the PCIM 52 is operational and monitoring the status of the system. The second time period may be immediately after the first time period, or (particularly in the case where the reference information is training data for the PCIM 52), the second time period may be some time after the first time period.

In embodiments where the reference information includes reference performance information, the operational information for the PCIM 52 can further comprise operational performance information indicating the operational reliability of the PCIM 52 in issuing status alerts for the system in the second time period. The operational performance information can comprise one or more of a true positive rate, a false positive rate, a true negative rate and a false negative rate. Alternatively the operational performance information can comprise predicted alert information which is information on alerts or issues predicted by the PCIM 52 in the second time period, and actual alert information which is information on actual alerts or issues raised by a user of the system in the second time period.

In step 807, a set of operational probability distributions are determined from the set of values obtained in step 805. The set of operational probability distributions comprise a respective operational probability distribution for each of the system features that is determined from the values of the respective system features in the second time period. The probability distributions can be determined as described above with reference to step 403 of Fig. 5 and step 504 of Fig. 6.

It will be appreciated that in some embodiments steps 801 and 803 may be performed some time before the second time period, and thus some time before the performance of the PCIM 52 is to be assessed. In other embodiments, steps 801-807 can be performed when the performance of the PCIM 52 is to be assessed.

Next, in step 809, a drift measure is determined for the PCIM 52 representing a measure of drift in performance of the PCIM 52 between the first time period and the second time period. The drift measure is based on a comparison of the set of reference probability distributions and the set of operational probability distributions. The drift measure can be determined as described above with reference to step 404 of Fig. 5 and steps 505-508 of Fig. 6.

In some embodiments step 809 comprises, for each system feature, comparing one or more statistical measures for the respective reference probability distribution to one or more statistical measures for the respective operational probability distribution. As noted above with regard to step 403 of Fig. 5 and step 504 of Fig. 6, the statistical measures can be any one or more of: a mean of the probability distribution, a standard deviation of the probability distribution, a density of the probability distribution, and one or more shape parameters defining the shape of the probability distribution. In some embodiments the statistical measures are compared by determining a distance measure for a system feature from the value of the statistical measure for the reference probability distribution for that system feature and the value of the statistical measure for the operational probability distribution for that system feature. The distance measure can be determined as described above with reference to equation (5).

In embodiments where the reference information and operational information include performance information, the drift measure determined in step 809 can be further based on a comparison of the reference performance information and the operational performance information (e.g. a comparison of the TP rate in the first time period and the TP rate in the second time period).

In step 811 the drift measure is output. For example the drift measure can be output to an operator or developer of the PCIM 52. In addition or alternatively, the drift measure can be output to a subsequent step in which the reasons for the drift are determined.

In some embodiments the method can also include obtaining values of one or more further features (meta features) relating to the system, with the further features comprising any of a presence of a log file for the system, a warranty status of a component of the system, or a version of software or firmware used by the system. This corresponds to step 406 in Fig. 5. In these embodiments the drift measure is further based on the values of the one or more further features, as described above with reference to Fig. 7.

In some embodiments, the PMM 40 does not initially have information about the PCIM 52 or the system being monitored by the PCIM 52, in which case the PMM 40 needs to determine the system features that are monitored by the PCIM 52 in order to obtain the reference information. Thus, prior to step 801, the method can further comprise analysing the PCIM 52 to identify the plurality of system features relating to the system that are used by the PCIM 52. This step can include analysing a computer file relating to the PCIM 52 to identify the system features used as inputs to the PCIM 52.

In some embodiments after step 809 the method further comprises evaluating the drift measure to identify one or more of the system features that have contributed to the value of the drift measure. This corresponds to step 508 of Fig. 6. The identified one or more system features that have contributed to the value of the drift measure can be analysed to determine corrections to the operation of the PCIM 52 to reduce the drift measure. This corresponds to step 407 of Fig. 5. In some embodiments the method can further comprise analysing the determined drift measure to estimate a remaining life of the PCIM 52.

There is therefore provided techniques for automatically monitoring the performance of predictive models without the need for a subject matter expert or other person to manually review the performance of the predictive model. In certain embodiments, it can be possible to identify and also implement appropriate corrections to the predictive model in case drift or deterioration in the performance of the predictive model is identified.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer-implemented method of monitoring performance of a predictive computer-implemented model, PCIM, that is used to monitor the status of a first system, wherein the PCIM receives as inputs observed values for a plurality of features relating to the first system, and the PCIM determines whether to issue status alerts based on the observed values, wherein the method comprises:

   obtaining reference information for the PCIM, wherein the reference information for the PCIM comprises a first set of values for the plurality of features relating to the first system in a first time period;

determining a set of reference probability distributions from the first set of values, the set of reference probability distributions comprising a respective reference probability distribution for each of the features that is determined from the values of the respective feature in the first set of values;

obtaining operational information for the PCIM, wherein the operational information for the PCIM comprises a second set of values for the plurality of features relating to the first system in a second time period that is after the first time period;

determining a set of operational probability distributions from the second set of values, the set of operational probability distributions comprising a respective operational probability distribution for each of the features that is determined from the values of the respective feature in the second set of values;

determining a drift measure for the PCIM representing a measure of drift in performance of the PCIM between the first time period and the second time period, wherein the drift measure is based on a comparison of the set of reference probability distributions and the set of operational probability distributions; and

output the drift measure.

2. A method as claimed in claim 1, wherein the step of determining the drift measure comprises, for each feature relating to the first system, comparing one or more statistical measures for the reference probability distribution of said feature to one or more statistical measures for the operational probability distribution of said feature.

3. A method as claimed in claim 2, wherein the step of comparing comprises, for each feature relating to the first system and for each statistical measure, determining a distance measure for said feature and statistical measure from the value of said statistical measure for the reference probability distribution and the value of said statistical measure for the operational probability distribution.

4. A method as claimed in claim 2 or 3, wherein the one or more statistical measures comprises any one or more of: a mean of the probability distribution, a standard deviation of the probability distribution, a density of the probability distribution, and one or more shape parameters defining the shape of the probability distribution.

5. A method as claimed in any of claims 1-4, wherein the first set of values for the plurality of features is a training set of values that was used to train the PCIM, and the first time period is a time period before the PCIM is monitoring the status of the first system.

6. A method as claimed in claim 5, wherein:

the reference information for the PCIM further comprises reference performance information indicating an expected reliability of the PCIM in issuing status alerts for the first system based on the training set of values;

the operational information for the PCIM further comprises operational performance information indicating the operational reliability of the PCIM in issuing status alerts for the first system in the second time period; and

the drift measure is further based on a comparison of the reference performance information and the operational performance information.

7. A method as claimed in any of claims 1-4, wherein the first set of values for the plurality of features is a set of values obtained during use of the PCIM, and the first time period is a time period where the PCIM is monitoring the status of the first system.

8. A method as claimed in claim 7, wherein:

the reference information for the PCIM further comprises reference performance information indicating the reliability of the PCIM in issuing status alerts for the first system in the first time period;

the operational information for the PCIM further comprises operational performance information indicating the operational reliability of the PCIM in issuing status alerts for the first system in the second time period; and

the drift measure is further based on a comparison of the reference performance information and the operational performance information.

9. A method as claimed in claim 6 or 8, wherein each of the reference performance information and the operational performance information comprise one or more of a true positive rate, a false positive rate, a true negative rate and a false negative rate.

10. A method as claimed in any of claims 1-9, wherein the method further comprises:

obtaining values of one or more further features relating to the first system, the one or more further features comprising any of a presence of a log file for the first system, a warranty status of a component of the first system, a version of software or firmware used by the first system; and

wherein the drift measure is further based on the values of the one or more further features.

11. A method as claimed in any of claims 1-10, wherein the method further comprises:
analysing the PCIM to identify the plurality of features relating to the first system that are used by the PCIM.

12. A method as claimed in any of claims 1-11, wherein the method further comprises:

evaluating the drift measure to identify one or more of the features that have contributed to the value of the drift measure; and

analysing the identified one or more features that have contributed to the value of the drift measure to determine corrections to the operation of the PCIM to reduce the drift measure.

13. A method as claimed in any of claims 1-12, wherein the method further comprises:
analysing the determined drift measure to estimate a remaining life of the PCIM.

14. A computer program product comprising a computer readable medium having computer readable code embodied therein, the computer readable code being configured such that, on execution by a suitable computer or processor, the computer or processor is caused to perform the method of any of claims 1-13.

15. An apparatus for monitoring performance of a predictive computer-implemented model, PCIM, that is used to monitor the status of a first system, wherein the PCIM receives as inputs observed values for a plurality of features relating to the first system, and the PCIM determines whether to issue status alerts based on the observed values, wherein the apparatus comprises a processing unit is configured to:

obtain reference information for the PCIM, wherein the reference information for the PCIM comprises a first set of values for the plurality of features relating to the first system in a first time period;

determine a set of reference probability distributions from the first set of values, the set of reference probability distributions comprising a respective reference probability distribution for each of the features that is determined from the values of the respective feature in the first set of values;

obtain operational information for the PCIM, wherein the operational information for the PCIM comprises a second set of values for the plurality of features relating to the first system in a second time period that is after the first time period;

determine a set of operational probability distributions from the second set of values, the set of operational probability distributions comprising a respective operational probability distribution for each of the features that is determined from the values of the respective feature in the second set of values;

determine a drift measure for the PCIM representing a measure of drift in performance of the PCIM between the first time period and the second time period, wherein the drift measure is based on a comparison of the set of reference probability distributions and the set of operational probability distributions; and

cause the output of the drift measure.

Fig. 1

Fig. 2

Fig. 3

Fig. 4

Fig. 5

EP 3 800 645 A1

501 — Predictive model

503 — Extract features from the model to create a pool of ground truth data

502 — Analyze the features of the model

504 — Compute the distribution of features

505 — Is distribution closer to GT?

506 — Model is performing as intended

507 — Find points of deviation from ground truth

508 — Identify possible cause of model drift

510 — Improve Model

509 — Alert

Fig. 6

70

72

56

74

Fig. 7

801

803

805

807

809

811

Fig. 8

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | AYSE KIVILCIM COSKUN ET AL: "Proactive temperature balancing for low cost thermal management in MPSoCs", COMPUTER-AIDED DESIGN, 2008. ICCAD 2008. IEEE/ACM INTERNATIONAL CONFERENCE ON, IEEE, PISCATAWAY, NJ, USA, 10 November 2008 (2008-11-10), pages 250-257, XP031363747, ISBN: 978-1-4244-2819-9 | 1-4,6-15 | INV. G16H50/20 |
| Y | * page BIBL/DESC 1 left col par 1, page BIBL/DESC 1 right col par 1, page DESC 1 right col last par - page DESC 2 left col par 6, page DESC 3 left col par 2 - page DESC 3 right col par 6 * ----- | 5 | |
| Y | RAHUL KHANNA ET AL: "System approach to intrusion detection using hidden Markov model", INTERNATIONAL WIRELESS COMMUNICATIONS AND MOBILE COMPUTING CONFERENCE : IWCMC 2006; JULY 3 - 6, 2006, VANCOUVER, BRITISH COLUMBIA, CANADA, NEW YORK, NY : ACM, 2 PENN PLAZA, SUITE 701 NEW YORK NY 10121-0701 USA, 3 July 2006 (2006-07-03), pages 349-354, XP058335481, DOI: 10.1145/1143549.1143619 ISBN: 978-1-59593-306-5 | 5 | |
| A | * page 351 left col 6th full par * ----- | 1-4,6-15 | TECHNICAL FIELDS SEARCHED (IPC)<br><br>G16H |

-/--

The present search report has been drawn up for all claims

| Place of search | Date of completion of the search | Examiner |
|---|---|---|
| Munich | 18 March 2020 | Bankwitz, Robert |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 19 20 1305

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | AHMED FAGHRAOUI ET AL: "SOA-based platform implementing a structural modelling for large-scale system fault detection: Application to a board machine", CONTROL APPLICATIONS (CCA), 2012 IEEE INTERNATIONAL CONFERENCE ON, IEEE, 3 October 2012 (2012-10-03), pages 681-685, XP032297969, DOI: 10.1109/CCA.2012.6402705 ISBN: 978-1-4673-4503-3 * abstract, page 682 right col par 2, page 684 left col last parpage 685 left col par 1 * | 1-15 | |
| A | CHIARA DI FRANCESCOMARINO ET AL: "Incremental Predictive Process Monitoring: How to Deal with the Variability of Real Environments", ARXIV.ORG, CORNELL UNIVERSITY LIBRARY, 201 OLIN LIBRARY CORNELL UNIVERSITY ITHACA, NY 14853, 11 April 2018 (2018-04-11), XP080869743, * abstract, page DESC 1 par 2 * | 1-15 | TECHNICAL FIELDS SEARCHED (IPC) |

The present search report has been drawn up for all claims

| Place of search | Date of completion of the search | Examiner |
|---|---|---|
| Munich | 18 March 2020 | Bankwitz, Robert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)